Europäisches Patentamt

European Patent Office

Office européen des brevets

(19)

(11) Veröffentlichungsnummer: **0 153 952 B1**

(12) # EUROPÄISCHE PATENTSCHRIFT

(45) Veröffentlichungstag der Patentschrift: 25.09.91

(51) Int. Cl.⁵: **A61M 5/00, A61B 5/04**

(21) Anmeldenummer: **84903346.9**

(22) Anmeldetag: **24.08.84**

(86) Internationale Anmeldenummer:
**PCT/EP84/00257**

(87) Internationale Veröffentlichungsnummer:
**WO 85/00982 (14.03.85 85/07)**

(54) **SPRITZE ZUM ANSCHLUSS AN VENENKATHETER ZUR LAGEKONTROLLE BEI DESSEN LEGEN UND GEBRAUCHEN.**

(30) Priorität: **26.08.83 DE 8324566 U**

(43) Veröffentlichungstag der Anmeldung:
**11.09.85 Patentblatt 85/37**

(45) Bekanntmachung des Hinweises auf die
Patenterteilung:
**25.09.91 Patentblatt 91/39**

(84) Benannte Vertragsstaaten:
**AT BE CH DE FR GB LI LU NL SE**

(56) Entgegenhaltungen:
**DE-U- 8 229 899**
**FR-A- 2 026 069**
**US-A- 2 646 042**
**US-A- 3 078 850**

(73) Patentinhaber: **Sterimed Gesellschaft für medizinischen Bedarf mbH
Fasanerieweg 15-17 Postfach 215
W-6600 Saarbrücken 3(DE)**

(72) Erfinder: **MÄRZ, Peter
Frauenschuhstrasse 31
W-8122 Penzberg(DE)**
Erfinder: **POSTEL, Jürgen
Hahnemannstrasse 2
W-8000 München 50(DE)**

(74) Vertreter: **RUPP, Herbert Byk Gulden Lomberg
Chemische Fabrik GmbH
Byk-Gulden-Strasse 2 Postfach 6500
W-7750 Konstanz(DE)**

## Beschreibung

Die Erfindung betrifft eine Spritze gemäß dem Oberbegriff der Ansprüche 1,2 und 3 zur Lagekontrolle eines Venenkatheters bei dessen Legen und Gebrauchen.

Eine Lagekontrolle eines Venenkatheters ist bereits bei dessen Legen erforderlich. Nachdem die Vene punktiert und der Katheter eingeführt worden ist, wird der Katheter unter laufender EKG-Kontrolle in Richtung Herz vorgeschoben, bis auf dem Monitor die Potentiale des Herzvorhofs erkennbar sind. Ein weiteres Vorschieben wäre gefährlich, da die Katheterspitze in den Ventrikel gelangen und hier Rhythmusstörungen auslösen könnte. Alsdann wird der Katheter ca. 2 bis 3 cm zurückgezogen. Dabei verschwinden die speziellen Vorhofpotentiale wieder und der Anwender weiß nun, daß die Katheterspitze vor dem Vorhof liegt. Dies ist die korrekte Lage eines zentralen Venenkatheters. Die bisher bekanntgewordenen Vorrichtungen zum Ableiten der EKG-Potentiale des Herzvorhofs haben sich hinsichtlich des Gebrauchs und der Herstellung als noch verbesserungsbedürftig erwiesen.

Für eine wirklich gute Brauchbarkeit sind an eine Vorrichtung zum Ableiten intracardialer EKG-Potentiale über einen zentralen Venenkatheter zum Zwecke der Lagekontrolle folgende Anforderungen zu stellen:
Herstellung als steriler Einmalartikel,
der Vorgang des Katheterlegens einschließlich der Messung muß unter vollständig sterilen Bedingungen möglich sein,
für spätere Kontrollen (Überprüfung der Katheterlage z.B. alle zwei Tage) soll der Meßvorgang an den bisher üblichen Infusionsleitungssystemen möglich sein. Ein Auseinanderschrauben der Infusionsleitung darf wegen der Gefahr der damit verbundenen Luftembolie nicht erforderlich werden.

Zwischen Infusionsleitung und Katheter sollte kein zusätzliches, bleibendes Teil eingebaut werden, da jede Konnektorstelle beim Dauerbetrieb die Gefahr einer unbeabsichtigten Lösung in sich birgt und insbesondere jede Verbindungsstelle eine Keimeintrittspforte darstellt.

Aus Gründen der Anwendungssicherheit sollte die Lagekontrolle während des Katheterlegens mit den gleichen Handgriffen durchführbar sein, wie der Vorgang des Katheterlegens selbst.

Durch die Anwendung des Geräts zur EKG-Lagekontrolle dürfen keine störenden Nebeneffekte auftreten, wie z.B. zusätzliche, herumhängende Kabel wären bei der Pflege des Patienten hinderlich und könnten zu einem unbeabsichtigten Herausreißen des Katheters führen,
zusätzliche in das Infusionssystem eingebrachte Teile (z.B. metallische Kontakte zur Potentialableitung) können den glatten, ungehinderten Durchfluß stören. Jede Unterbrechung der glatten Oberfläche beinhaltet die Gefahr einer Blutgerinnselbildung (über den Katheter werden ja auch Bluttransfusionen verabreicht und es wird ebenso Blut über den Katheter zu diagnostischen Zwecken abgenommen). Diese Gerinnsel können einerseits durch den Katheter zum Herzen weitergespült werden, sich andererseits aber auch in feinsten Ritzen festsetzen und so eine hervorragende Brutstätte für Bakterien bilden.

Bei der Blutabnahme dürfen Laborwerte nicht verfälscht werden (z.B. Eisen, Kupfer).

Aus Kostengründen muß das Gerät in einfachster Bauweise herstellbar sein.

Diesen komplexen Anforderungen sind die bisher bekanntgewordenen Geräte noch nicht ausreichend gewachsen.

Die aus dem DE-GM 82 29 899 bekannte Zwischenschaltung eines isolierten Adapters mit elektrischer Anschlußleitung zwischen Infusionsleitung und Katheter kann in seiner Ausführung zwar einfach gestaltet werden, bedingt aber das Problem des Auseinanderbauens der Infusionsleitung mit den damit verbundenen Komplikationsmöglichkeiten. Ein solches Auseinanderbauen erfordert insbesondere verschiedene Handgriffe und kann auch Probleme hinsichtlich der Sterilität und einer Auslaufgefahr für die Infusionsflüssigkeit mit sich bringen.

Der an eine Spritze anschließbare Adapter gemäß Literaturstelle Anästhesia and Analgesia, Vol. 61, No. 10, Oktober 1982, Seite 884 ("New Adapter for Intravascular Electrocardiogram") enthält ein Schlauchsystem mit innenliegenden Drähten als Kontaktteil läßt nach Angabe der Autoren die Verwendung heparinisierter Lösungen empfehlenswert erscheinen, um ungehinderten Blutrückfluß bei dem Lagekontrollvorgang nach dem Legen des Katheters zu gewährleisten. Auch führt die Verlängerung des elektrisch leitenden Systems zwischen Katheter und EKG-Kabel zu einer Antennenwirkung. Hier werden Störsignale empfangen, die dann die abgeleitete EKG-Kurve auf dem Monitor überlagern.

Der vorliegenden Erfindung liegt die Aufgabe zugrunde, eine Spritze zum Anschluß an Venenkatheter zu deren Lagekontrolle beim Legen und Gebrauchen der Venenkatheter dahingehend zu verbessern, daß sie in optimaler Weise den oben herausgestellten komplexen Anforderungen genügt.

Ausgehend von einer Spritze gemäß dem Oberbegriff sind in den Ansprüchen 1, 2 und 3 drei Verschiedene Lösungen dieser Aufgabe gekennzeichnet, die jeweils dem gleichen Prinzip folgen.

In jedem Falle stellt die Erfindung sicher, daß die für den Ablauf der Lagekontrolle erforderlichen Teile dem Anwender steril zur Verfügung stehen und daß der sterile Bereich nicht durch von aussen zugeführte unsterile Teile unterbrochen wird.

Die erfindungsgemäße Ausgestaltung der Spritze stellt aber auch sicher, daß über den Venenkatheter leicht und ungehindert Blut aspirierbar ist. Der Anwender kann also mit Sicherheit vermeiden, daß der Katheter mit der Spitze an der Venenwand anliegt oder sich in einem kleinen Seitenast verfängt. Intimaschäden an der Venenwand mit nachfolgender Thrombosierung sind damit ausgeschlossen.

Während bisher zur EKG-Lagekontrolle spezielle Elektrolytlösungen, insbesondere Kochsalzlösung empfohlen wurden, haben vergleichende Leitfähigkeitsmessungen der verschiedensten Infusionslösungen gezeigt, daß neben praktisch allen elektrolythaltigen Infusionslösungen ebensogut Blut geeignet ist. Das zur Kontrolle des Blutrückflusses angesaugte Blut kann also gleichzeitig zur Ableitung der intrakardialen Potentiale ausgenutzt werden. Die Spritze nach der vorliegenden Erfindung gestattet die Ausnutzung dieses Effektes ohne eine Thrombosierungsgefahr und andere Nachteile.

Während mit der Spritze Blut angesaugt wird, um den Rückfluß zu testen, werden gleichzeitig die EKG-Potentiale von der Spitze des Katheters abgeleitet und auf dem Monitor sichtbar, d.h. die Lagekontrolle kann exakt mit den gleichen Handgriffen durchgeführt werden, die bisher auch beim Legen des Katheters angewendet wurden.

Die spätere Lagekontrolle wird in ebenso einfacher Weise durchgeführt. Über den freien Weg des Dreiwegehahns, an dem üblicherweise die Blutentnahme zu Laborkontrollen erfolgt, wird mit der Spritze nach der Erfindung ebenfalls Blut aspiriert, so daß über die Blutsäule die EKG-Potentiale von der Spitze des Katheters abgeleitet werden können. Da bei diesem Vorgang üblicherweise auch der dritte Weg in Richtung Infusionsleistung verschlossen ist, ist so ein optimaler Schutz gegen Störenflüsse im Sinne einer Antennenwirkung gegeben.

Das Gerät kann als preiswerter Einmalartikel hergestellt werden, da eben gerade wegen dieses Einmalgebrauchs keine besonderen Anforderungen an die Ausführungen des Kontaktteils zu stellen sind.

Da die zum Anschluß an das EKG-Gerät dienende Leitung direkt an der Spritze befestigt ist, ist das Katheterlegen sowie die gleichzeitig durchgeführte Lagekontrolle unter völlig sterilen Bedingungen möglich, d.h. es muß keinerlei unsteriles Teil von außen in den sterilen Arbeitsbereich herangeführt werden.

Wegen seiner Kleinheit und einfachen Bauweise kann das gesamte Gerät in einem Katheterset steril mitverpackt werden, so daß jetzt der gesamte Vorgang des Katheterlegens einschließlich seiner Lagekontrolle von einer Person ausgeführt werden kann. Das sterile Anreichen irgendwelcher zusätzlicher Teile durch eine Hilfsperson entfällt.

Zum Stande der Technik ist noch zu erwähnen, daß es an sich der ständigen Praxis entspricht, in der Verbindungsleitung zwischen Venenkatheter und Infusionsbehälter einen Dreiwegehahn vorzusehen. Mit der vorliegenden Erfindung läßt sich diese bekannte Anordnung auf besonders günstige Weise für die Lagekontrolle eines Venenkatheters mittels einer mit elektrischem Kontaktteil ausgestatteten Spritze ausnutzen.

Es folgt die Beschreibung von Ausführungsbeispielen der Erfindung anhand einer Zeichnung. Diese zeigt eine Vorrichtung zur Katheterisierung von Venen mit einer Spritze mit angeschlossener, zu einem EKG-Gerät führender Leitung, sowie mit einem Dreiwegehahn, der einen Venenkatheter, einen Infusionsschlauch und die Spritze miteinander verbindet.

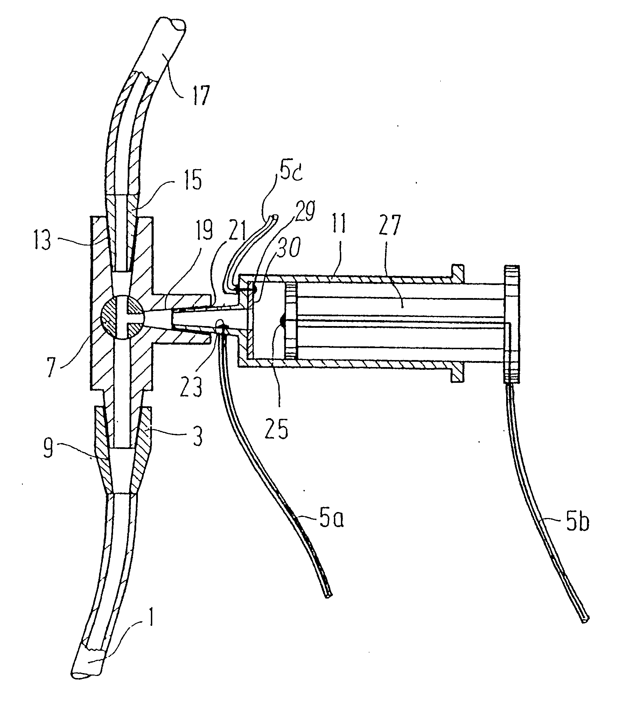

In der Figur sieht man einen Venenkatheter 1 üblicher Art, der einen weiblichen Anschlußkonus 3 aufweist.

Bei dem Venenkatheter handelt es sich beispielsweise um einen dünnen Kunststoffschlauch, der nach Punktierung der Vene in bekannter Weise in diese eingeführt und mit zum Herzen vorgeschoben wird. Zur Lagekontrolle der Spitze des Venenkatheters beim Eintritt in das Herz wird der Venenkatheter in bekannter Weise mit Elektrolytflüssigkeit aufgefüllt, und in ebenfalls bekannter Weise werden dann die durch die Elektrolytflüssigkeit geleiteten elektrischen Herzimpulse über Anschlußleitungen zu einem EKG-Gerät geleitet.

Der weibliche Anschlußkonus 3 des Venenkatheters entspricht der genormten Ausführung, so daß nach Bedarf ein in schematisch gezeigter Dreiwegehahn 7 mit genormtem männlichen Anschlußkonus 9 oder aber auch unmittelbar eine Injektionsspritze der bei 11 mit ihrem Spritzen Körper gezeigten Art angeschlossen werden kann. Von den drei Wegen des Dreiwegehahns 7 ist der eine weibliche Anschlußkonus 13 an den männlichen Anschlußkonus 15 eines von einem Infusionsbehälter herkommenden Infusionsschlauchs 17 angeschlossen, und der weibliche Anschlußkonus 19 des dritten Wegs des Dreiwegehahns 7 ist an den männlichen Anschlußkonus 21 der Spritze angeschlossen, die mit einem elektrischen Kontaktteil ausgestattet ist.

Zu diesem Zweck kann die elektrische Anschlußleitung 5a in der dargestellten Weise in dem Flüssigkeitsraum der Spritze mit einem Kontaktteil 23 an der Innenseite des Spritzenzylinders münden, oder es kann die elektrische Anschlußleitung 5b mit einem Kontaktteil 25 an dem inneren Ende des Spritzenkolbens 27 münden.

Gemäß einer weiteren Ausführungsmöglichkeit, die auch in der Figur veranschaulicht ist, kann eine elektrische Anschlußleitung 5c für den Anschluß an das EKG-Gerät ein Kontaktteil 29 aufweisen, welches mit einer Metallplatte 30 am Vorderende des Innenraums der Spritze vernietet ist.

In jedem Falle bilden die Spritze, das Kontaktteil und die Anschlußleitung für das EKG-Gerät eine sterile Einheit, welche aufgrund der Unabhängigkeit von anderen Teilen eine besonders griffgünstige Durchführung des Lagekontrollvorgangs gestaltet und dem Anwender auf einfache Weise in steriler Form zur Verfügung gestellt werden kann. Das wird auch durch die Flexibilität der Anschlußleitung erleichtert.

Mittels des Dreiwegehahns 7 ist die Flüssigkeits-Verbindungsleitung zwischen Venenkatheter 1 und dem vom Infusionsbehälter herkommenden Infusionsschlauch 17 unterbrechbar und statt dessen die Spritze anschließbar, die mit dem elektrischen Kontaktteil 23 o.dgl. und der Anschlußleitung 5a o.dgl. für das EKG-Gerät ausgestattet ist. Bei der Spritze kann es sich um die gleiche Spritze handeln, die normalerweise beim Legen eines Venenkatheters gebraucht wird, um durch Ansaugen von Blut festzustellen, ob die Vene punktiert wurde und der Venenkatheter in die Vene eintreten kann.

Für den Lagekontrollvorgang ist dann die Flüssigkeitsverbindungsleitung zwischen Venenkatheter 1 und Spritze mittels des Dreiwegeventils von dem Infusionsbehälter trennbar. Das ist auch dann bedeutsam, wenn die Infusionsflüssigkeit nicht elektrolytisch ist und mittels der Spritze dann Elektrolytflüssigkeit in den Venenkatheter eingespritzt werden kann.

Für den Fall, daß keine größeren Mengen Infusionsflüssigkeit oder keine elektrolytisch-leitende Infusionsflüssigkeit angewendet werden müssen, kann die Spritze mit ihrem Anschlußkonus 21 auch unmittelbar an den Anschlußkonus 3 des Venenkatheters 1 angeschossen werden, um die elektrolytische Flüssigkeit in dem Venenkatheter über die elektrische Anschlußleitung 5a o.dgl. zur Lagekontrolle des Venenkatheters an das EKG-Gerät anzuschließen.

## Patentansprüche

1. Elektrisch isolierende Spritze zum Anschluß an Venenkatheter für das Ansaugen und Ausspritzen von Flüssigkeiten und mit Anschlußmöglichkeit an eine zu dem EKG-Gerät führende Leitung, mittels deren das an der Flüssigkeitssäule in dem Venenkatheter anliegende elektrische Potential zur Lagekontrolle des Venenkatheters bei dessen Legen und Gebrauchen meßbar ist, wobei die Spritze (6) aus einem Spritzen-Kolben (27), einem Spritzen-Körper (11) und einem Anschlußkonus (21) sowie einem Kontaktteil (23) für die zum EKG-Gerät führende Leitung (5a) besteht,

**dadurch gekennzeichnet,**
daß Spritzen-Körper (11), Anschlußkonus (21) und das im Anschlußkonus (21) mündende Kontaktteil (23) unlösbar verbunden und mit der zum Anschluß an das EKG-Gerät dienenden Leitung (5a) sowie dem Spritzenkolben (27) steril sind.

2. Elektrisch isolierende Spritze zum Anschluß an Venenkatheter für das Ansaugen und Ausspritzen von Flüssigkeiten und mit Anschlußmöglichkeit an eine zu dem EKG-Gerät führende Leitung, mittels deren das an der Flüssigkeitssäule in dem Venenkatheter anliegende elektrische Potential zur Lagekontrolle des Venenkatheters bei dessen Legen und Gebrauchen meßbar ist, wobei die Spritze (6) aus einem Spritzen-Kolben (27), einem Spritzen-Körper (11) und einem Anschlußkonus (21) sowie einem Kontaktteil (29) für die zum EKG-Gerät führende Leitung (5c) besteht,
**dadurch gekennzeichnet,**
daß der Spritzen-Körper (11), der Anschlußkonus (21) und das im Spritzen-Körper (11) mündende Kontaktteil (29) unlösbar verbunden und mit der zum Anschluß an das EKG-Gerät dienenden Leitung (5c) sowie dem Spritzenkolben steril sind.

3. Elektrisch isolierende Spritze zum Anschluß an Venenkatheter für das Ansaugen und Ausspritzen von Flüssigkeiten und mit Anschlußmöglichkeit an eine zu dem EKG-Gerät führende Leitung, mittels deren das an der Flüssigkeitssäule in dem Venenkatheter anliegende elektrische Potential zur Lagekontrolle des Venenkatheters bei dessen Legen und Gebrauchen meßbar ist, wobei die Spritze (6) aus einem Spritzen-Kolben (27), einem Spritzen-Körper (11) und einem Anschlußkonus (21) sowie einem Kontaktteil (25) für die zum EKG-Gerät führende Leitung (5b) besteht,
**dadurch gekennzeichnet,**
daß die Anschlußleitung (5b) mit ihrem Kontaktteil (25) an dem inneren Ende des Spritzen-Kolbens (27) mündet und damit unlösbar verbunden ist, wobei Spritzen-Körper (11), Anschlußkonus (21), Spritzen-Kolben (27) und elektrische Leitung (5b) steril sind.

## Claims

1. Electrically insulating syringe for connection to vein catheters for drawing off liquids by suction and ejecting liquids in a jet-like manner, and with a facility permitting connection to a lead which runs to the ECG apparatus and makes it possible to measure the electrical potential at the liquid column in the vein catheter for the purpose of checking the position of the vein catheter during its insertion and use, the syringe (6) consisting of a syringe plunger (27), a syringe body (11), a connection cone (21) and a contact piece (23) for the lead (5a) which runs to the ECG apparatus, characterized in that the syringe body (11), the connection cone (21) and the contact piece (23), which enters at a location inside the connection cone (21), are inseparably connected and, with the syringe plunger (27) and the lead (5a) which serves as the connection to the ECG apparatus, are sterile.

2. Electrically insulating syringe for connection to vein catheters for drawing off liquids by suction and ejecting liquids in a jet-like manner, and with a facility permitting connection to a lead which runs to the ECG apparatus and makes it possible to measure the electrical potential at the liquid column in the vein catheter for the purpose of checking the position of the vein catheter during its insertion and use, the syringe (6) consisting of a syringe plunger (27), a syringe body (11), a connection cone (21) and a contact piece (29) for the lead (5c) which runs to the ECG apparatus, characterized in that the syringe body (11), the connection cone (21) and the contact piece (29), which enters at a location inside the syringe body (11), are inseparably connected and, with the syringe plunger and the lead (5c) which serves as the connection to the ECG apparatus, are sterile.

3. Electrically insulating syringe for connection to vein catheters for drawing off liquids by suction and ejecting liquids in a jet-like manner, and with a facility permitting connection to a lead which runs to the ECG apparatus and makes it possible to measure the electrical potential at the liquid column in the vein catheter for the purpose of checking the position of the vein catheter during its insertion and use, the syringe (6) consisting of a syringe plunger (27), a syringe body (11), a connection cone (21) and a contact piece (25) for the lead (5b) which runs to the ECG apparatus, characterized in that the connecting lead (5b) enters with its contact piece (25) at the inner end of the syringe plunger (27) and is inseparably connected to it, the syringe body (11), connection cone (21), syringe plunger (27) and electrical lead (5b) being sterile.

**Revendications**

1. Seringue diélectrique à raccorder à un cathéter pour veines pour aspirer et injecter des liquides et présentant une possibilité de raccordement à une conduite menant à un électrocardiographe, au moyen de laquelle le potentiel électrique appliqué sur la colonne de liquide dans le cathéther pour veines peut être mesuré pour le contrôle de position dudit cathéter pour veines lors de sa mise en place et de son utilisation, la seringue (6) étant constituée d'un piston (27), d'un corps (11) et d'un cône de raccordement (21), ainsi que d'un élément de contact (23) pour la conduite (5a) menant à l'électrocardiographe, caractérisée en ce que le corps de seringue (11), le cône de raccordement (21) et l'élément de contact (23) débouchant dans le cône de raccordement (21) sont reliés de manière indétachable et sont aseptiques vis-à-vis de la conduite (5a) servant au raccordement à l'électrocardiographe, ainsi que vis-à-vis du piston de seringue (27).

2. Seringue diélectrique à raccorder à un cathéter pour veines pour aspirer et injecter des liquides et présentant une possibilité de raccordement à une conduite menant à un électrocardiographe, au moyen de laquelle le potentiel électrique appliqué sur la colonne de liquide dans le cathéther pour veines peut être mesuré pour le contrôle de position dudit cathéter pour veines lors de sa mise en place et de son utilisation, la seringue (6) étant constituée d'un piston (27), d'un corps (11) et d'un cône de raccordement (21), ainsi que d'un élément de contact (29) pour la conduite (5c) menant à l'électrocardiographe, caractérisée en ce que le corps de seringue (11), le cône de raccordement (21) et l'élément de contact (29) débouchant dans le corps de seringue (11) sont reliés de manière indétachable et sont aseptiques vis-à-vis de la conduite (5c) servant au raccordement à l'électrocardiographe, ainsi que vis-à-vis du piston de seringue.

3. Seringue diélectrique à raccorder à un cathéter pour veines pour aspirer et injecter des liquides et présentant une possibilité de raccordement à une conduite menant à un électrocardiographe, au moyen de laquelle le potentiel électrique appliqué sur la colonne de liquide dans le cathéther pour veines peut être mesuré pour le contrôle de position dudit cathéter pour veines lors de sa mise en place et de son utilisation, la seringue (6) étant constituée d'un piston (27), d'un corps (11) et d'un cône de raccordement (21), ainsi que d'un élément de contact (25) pour la conduite (5b) menant à l'électrocardiographe, caractérisée en ce que la conduite de raccordement (5b) débouche par son élément de contact (25) dans l'extrémité interne du piston de seringue (27) et, par suite, y est raccordée de manière indétachable, le corps de seringue (11), le cône de raccordement (21), le piston de seringue (27) et la conduite électrique (5) étant aseptiques.